# EUROPÄISCHE PATENTSCHRIFT

(11) **EP 0 472 708 B1**
(45) Veröffentlichungstag und Bekanntmachung des Hinweises auf die Patenterteilung: **24.08.1994**
(21) Anmeldenummer: 91906410.5
(22) Anmeldetag: 16.03.1991
(51) Int. Cl.: A61K 35/16, A61L 2/18, C07K 3/20, B01D 15/08

(54) **VERFAHREN ZUR HERSTELLUNG VON NICHT-INFEKTIÖSEM BLUTPLASMA**
PROCESS FOR PRODUCING NON-INFECTIOUS BLOOD PLASMA
PROCEDE POUR LA FABRICATION DE PLASMA SANGUIN NON-INFECTIEUX

(30) Priorität: 20.03.1990 DE 4008852
(43) Veröffentlichungstag der Anmeldung: 04.03.1992
(73) Patentinhaber: New York Blood Center, Inc., New York, New York 10021 (US)
(72) Erfinder: WOLTER, Dieter, D-5800 Hagen (DE); SCHWINN, Horst, D-3550 Marburg (DE)
(74) Vertreter: Jaenichen, Hans-Rainer, Dr.
(86) Internationale Anmeldenummer: EP9100503
(87) Internationale Veröffentlichungsnummer: WO9114439

(56) Entgegenhaltungen:
- EP-A- 0 047 462
- EP-A- 0 050 061
- EP-A- 0 366 946
- EP-A- 0 367 840
- US-A- 4 591 505
- Toxical and Appl. Pharmacol. 1969 vol 14 pp315-334
- US 4,412,985
- Concise Chem. Tech. Dict.
- Römpp Chemie Lexikon
- Chemical Abstracts, Band 111, Nr. 3, 17.07.89 Seite 281

## Beschreibung

Gegenstand der vorliegenden Erfindung ist ein Verfahren zur Herstellung von nicht-infektiösem Blutplasma.

Blutplasma wird in der Medizin für eine Fülle von Indikationen verwendet. Entsprechend groß ist seine klinische Bedeutung. Das Blutplasma wird durch Abtrennung der Blutzellen aus Vollblut, das Spendern entnommen worden ist, gewonnen. Um Infektionen des Patienten mit pathogenen Bestandteilen, wie Hepatitisviren und HIV des Spenderplasmas zu vermeiden, muß das Plasma frei von intakten Viren sein. Deshalb war es bislang nur möglich, Plasmen von einzelnen Spendern einzusetzen, die nach einer längeren Beobachtungszeit als gesund im Sinne von Virusfreiheit betrachtet werden konnten. Ein Vermischen (Poolen) von Plasmen mehrerer Spender war ausgeschlossen.

Nachteilig an dieser Verfahrensweise ist der enorme Aufwand, der durch die einzelnen Präparationen aufgewendet werden muß. Eine kontinuierliche Arbeitsweise ist mit den derzeit bestehenden Arbeitstechniken nicht möglich. Bedingt dadurch tritt ein weiterer Nachteil in Erscheinung. Es ist nur mit unverhältnismäßig hohem, kaum vertretbarem Aufwand möglich, standardisierte Plasmapräparationen herzustellen. Bedingt durch die Einzelzubereitung schwankt beispielsweise der Proteingehalt in der Spenderfraktion. Daher muß bei Verwendung des durch Einzelzubereitung hergestellten Plasmas jeweils der aktuelle Proteingehalt der betreffenden Charge berücksichtigt werden.

Ein großes Problem stellt die Entfernung des zur Inaktivierung der infektiösen Bestandteile verwendeten Mittels dar. Nach einer vom New-York-Blood-Center entwickelten Verfahrensweise werden zur Virus-Inaktivierung Detergenzien verwendet. Ein großes Problem besteht darin, diese Detergenzien nach erfolgter Virus-Inaktivierung dem Plasma wieder zu entziehen. Horowitz schlägt vor, die mit Detergenzien behandelten Zubereitungen mittels hydrophober Chromatographie durch Umkehrphasenmaterialien vom Detergenz zu befreien.

Das der Erfindung zugrunde liegende technische Problem ist somit die Bereitstellung eines kontinuierlichen Verfahrens zur Herstellung eines nicht-infektiösen Blutplasmas. Die sich daraus ergebenden Probleme betreffen unter anderem eine sichere und zuverlässige Verfahrensweise zur Entfernung der virus-inaktivierenden Detergenzien. Darüber hinaus ist es wünschenswert, bereits bei der Herstellung der Plasmapräparate solche mit standardisiertem Proteingehalt zu erhalten.

Die der Erfindung zugrunde liegenden technischen Probleme werden gelöst durch ein Verfahren zur Herstellung von nicht-infektiösem Blutplasma, wobei das Plasma mit nicht-ionischen Tensiden behandelt wird, biologisch kompatible Lipide Zugesetzt werden, die Lipidphase abgetrennt wird und die nicht-ionischen Tenside durch Festphasenextraktion an hydrophoben Materialien entfernt werden.

Das Blutplasma kann dabei sowohl von frisch hergestellten Plasmapräparationen stammen als auch von tiefgefrorenen Fraktionen. Dabei wird gefrorenes Plasma in einem Wasserbad aufgetaut. Sobald eine gewisse Temperatur, die vorzugsweise 25°C beträgt, erreicht ist, wird das Plasma gegebenenfalls filtriert und der pH-Wert der Lösung kontrolliert. Der Auftauvorgang wird vorzugsweise in einer Zeit von etwa 1 bis 2 Stunden durchgeführt. Der pH-Wert der Lösung beträgt vorzugsweise 7,0 bis 7,4.

Danach wird das so behandelte Blutplasma mit einer Mischung aus nicht-ionischen Tensiden und destilliertem Wasser bei 20 bis 40°C, vorzugsweise 30°C, behandelt. Als nicht-ionische Tenside kommen insbesondere Tri-N-Butylphosphat (TNBP) und/oder Triton® X-100 in Frage. Es ist vorteilhaft, die Mischung unter leichtem Rühren einige Zeit bei erhöhter Temperatur zu behandeln. Anschließend wird die Lipidfraktion durch Zentrifugation entfernt. Es ist vorteilhaft, vor dem Zentrifugationsschritt die Lipidfraktion mit biologisch verträglichen Lipiden, wie Pflanzenöl, insbesondere Sojaöl und/oder Rizinusöl, anzureichern. In einer bevorzugten Ausführungsform des erfindungsgemäßen Verfahrens werden dabei ca. 10 % Pflanzenöl zugefügt. Nach der Zentrifugation werden Lipid- und Plasmaschicht getrennt. Vorzugsweise kann dies mittels eines Siphons oder einer kontinuierlich arbeitenden Zentrifuge mit automatischer Phasentrennung geschehen.

Die Plasmaschicht wird gesammelt, während die Lipidschicht verworfen wird. Danach schließt sich gegebenenfalls ein Filtrationsschritt an, woraufhin das restliche noch vorhandene nicht-ionische Detergenz aus dem Plasma mittels Festphasenextraktion beseitigt wird. Vorzugsweise ist das hydrophobe Material zur Festphasenextraktion das aus der Umkehrphasen-Chromatographie (reversed phase chromatography) bekannte Material. Dazu gehören vorzugsweise mit C-18 beschichtete Trägermaterialien, wie sie auch in der Hochdruckflüssigkeits-Chromatographie Verwendung finden. In einer bevorzugten Ausführungsform kann die Festphasenextraktion in Form einer Chromatographie erfolgen. Das Verhältnis zwischen Bettvolumen und dem aufzuarbeitenden Plasmavolumen beträgt vorzugsweise 1 : 6 bis 1 : 20, besonders bevorzugt 1 : 10.

Nach der Festphasenextraktion schließt sich gegebenenfalls eine Kontrolle des pH-Wertes an. Der pH-Wert der Lösung wird auf pH 7,0 bis 7,4 nachjustiert. Danach schließt sich eine Sterilfiltration an, woraufhin gewünschtenfalls das Blutplasma gefriergetrocknet wird in Gegenwart üblicher Lyophilisations-Hilfsmittel, wie Lysin oder Glycin.

Das erfindungsgemäße Verfahren ermöglicht es, von der üblichen Einzelzubereitung des Plasmas abzugehen. Das Verfahren ist geeignet, im industriellen Maßstab durchgeführt zu werden. Insbesondere wird eine kontinuierliche Verfahrensweise ermöglicht. Ein weiterer Vorteil des erfindungsgemäßen Verfahrens liegt darin, daß aufgrund der kontinuierlichen Verfahrensgestaltung die Bedingungen so gewählt werden können, daß das Endprodukt jeweils gleiche oder zumindest sehr ähnliche Proteinkonzentrationen aufweist. Dies macht die klinische Handhabung der Blutplasma-Präparate, insbesondere bei Mehrfachgabe, unproblematischer, da die wechselnde Proteinmenge, die sich aus den Einzelpräparationen ergibt, entfällt.

Das erfindungsgemäße Verfahren wird anhand des folgenden Ausführungsbeispiels näher erläutert:
20 l Blutplasma (80 x 250 ml) werden im Wasserbad bei 25°C für die Dauer von 1,5 Stunden aufgetaut. Gewünschtenfalls wird die Fraktion nach dem Auftauen über Gazefilter oder Filterpatronen (z.B. Firma Pal, 100 »m) filtriert. Sodann wird das Plasma in einen 50 l Edelstahlkessel bei 30°C gefüllt. Für Rühroperationen wird ein Propeller-Blattrührer verwendet. Der pH-Wert des aufgetauten Plasmas wird überprüft und mit 0,5 N-Salzsäure auf einen Wert von pH 7,0 bis 7,4 eingestellt.

Es wird eine Mischung von TNBP, Triton® X-100 und destilliertem Wasser hergestellt. Die Mengen betragen vorzugsweise TNBP : Triton® X-100 : destilliertes Wasser = 0,5 bis 2 : 0,5 bis 2 : 2 bis 4. Bei der vorliegenden Ansatzgröße wird in besonders bevorzugter Weise 200 ml TNBP, 200 ml Triton® X-100 und 800 ml destilliertes Wasser verwendet. Die Mischung wird ebenfalls auf ca. 30°C erwärmt. Danach fügt man die Mischung der nicht-ionischen Tenside dem Plasma hinzu. Die Mischung wird bei 30°C vorsichtig gerührt. Nach 4 Stunden schließt sich die Zentrifugation des Plasmas an. Vorzugsweise wird mit 4.000 Umdrehungen pro Minute eine Stunde lang bei 12°C zentrifugiert. Es empfiehlt sich, zur Vergrößerung der Lipidphase etwa 10 % (v/v) Soja- oder Rizinusöl der Reaktionsmischung vor der Zentrifugation hinzuzugeben. Nach der Zentrifugation werden Lipid- und Plasmaschicht getrennt. Dies kann vermittels eines Siphon geschehen. Die Plasmaschichten werden gesammelt und mit Protein-inerten Filtermembranen (z.B. Firma Pal, 3 - 5 »m) filtriert. Man erhält 19 l Plasma.

Diese Menge wird mit "C-18 präparativ" Adsorbens (Waters, Inc.) behandelt. Bei der angefallenen Menge von 19 l werden vorzugsweise 1,2 kg in 2,4 l Wasser aufgeschlämmt. Die Chromatographie wird an einer Säule mit 15 cm Durchmesser durchgeführt. Die Fließrate beträgt 300 ml/min. Das Eluat wird mittels eines UV-Monitors überwacht. Die austretende Plasmafraktion wird gesammelt und gegebenenfalls auf einen pH von 7,0 bis 7,4 mit 0,5 N-Natronlauge eingestellt.

Das Plasma wird über Membranfilter (0,22 »m) steril filtriert und gefriergetrocknet, nachdem es in 250 ml Flaschen abgefüllt wurde.

## Patentansprüche

1. Verfahren zur Herstellung von nicht-infektiösem Blutplasma, wobei das Plasma mit nicht-ionischen Tensiden behandelt wird, biologisch kompatible Lipide Zugesetzt werden die Lipidphase abgetrennt wird und die nicht-ionischen Tenside durch Festphasenextraktion an hydrophoben Materialien entfernt werden.

2. Verfahren nach Anspruch 1, wobei das tiefgefrorene Blutplasma im Wasserbad bei 20 bis 30°C schonend aufgetaut wird.

3. Verfahren nach einem der Ansprüche 1 oder 2, wobei der pH-Wert des Plasmas auf pH 7,0 bis 7,4 eingestellt wird.

4. Verfahren nach einem der Ansprüche 1 bis 3, wobei als nicht-ionische Tenside Tri-N-Butylphosphat (TNBP) und/oder Triton® X-100 eingesetzt werden.

5. Verfahren nach einem der Ansprüche 1 bis 4, wobei eine wäßrige Mischung von TNBP und Triton® X-100 eingesetzt wird.

6. Verfahren nach Anspruch 5, wobei das Mischungsverhältnis von TNBP : Triton® X-100 : Wasser = 0,5 bis 2 : 0,5 bis 2 : 2 bis 4 ist.

7. Verfahren nach einem der Ansprüche 1 bis 6, wobei die Lipidphase durch Zentrifugation abgetrennt wird.

8. Verfahren nach einem der Ansprüche 1 bis 7, wobei die Lipidmenge ca. 10 % (v/v) beträgt.

9. Verfahren nach einem der Ansprüche 1 bis 8, wobei die Festphasenextraktion mit Materialien für die Umkehrphasen-Chromatographie (reversed phase) durchgeführt wird.

10. Verfahren nach einem der Ansprüche 1 bis 9, wobei Octadecyl derivatisierte Materialien für die Umkehrphasen-Chromatographie Verwendung finden.

11. Verfahren nach einem der Ansprüche 1 bis 9, wobei die Festphasenextraktion durch C-18 Umkehrphasen-Chromatographie erfolgt.

12. Verfahren nach einem der Ansprüche 1 bis 11, wobei auf die Festphasenextraktion gegebenenfalls nach Kontrolle des pH-Wertes und Justierung auf pH 7,0 bis 7,4 eine Sterilfiltration durchgeführt wird.

13. Verfahren nach einem der Ansprüche 1 bis 11, wobei das Blutplasma gefriergetrocknet wird gegebenenfalls in Gegenwart üblicher Hilfsmittel zur Lyophilisierung.

## Claims

1. A method for producing non-infectious blood plasma, comprising the steps of treating the plasma with non-ionic surfactants, adding biologically compatible lipids, separating the lipid phase and removing the non-ionic surfactants by solid-phase extraction on hydrophobic material.

2. The method according to claim 1 wherein the deep-frozen blood plasma is slowly thawed in a water bath at a temperature of 20 to 30°C.

3. The method according to claim 1 or 2 wherein the pH of the plasma is adjusted to pH 7.0 to 7.4.

4. The method according to any of claims 1 to 3 wherein tri-N-butyl phosphate (TNBP) and/or Triton® X-100 are used as non-ionic surfactants.

5. The method according to any of claims 1 to 4 wherein an aqueous mixture of TNBP and Triton® X-100 is used.

6. The method according to claim 5 wherein the ratio of TNBP:Triton® X-100:water is 0.5 to 2:0.5 to 2:2 to 4.

7. The method according to any of claims 1 to 6 wherein the lipid phase is removed by centrifugation.

8. The method according to any of claims 1 to 7 wherein the amount of lipids is about 10 % (v/v).

9. The method according to any of claims 1 to 8 wherein the solid-phase extraction is effected with materials for reversed-phase chromatography.

10. The method according to any of claims 1 to 9 wherein octadecyl-derivatised materials are used in the reversed-phase chromatography.

11. The method according to any of claims 1 to 9 wherein the solid-phase extraction is effected by C-18 reversed-phase chromatography.

12. The method according to any of claims 1 to 11 wherein, optionally, subsequent to the solid-phase extraction, a sterile filtration is carried out upon control of the pH and adjustment of the pH to 7.0 to 7.4.

13. The method according to any of claims 1 to 11 wherein the blood plasma is freeze-dried, optionally in the presence of conventional auxiliary agents for lyophilization.

## Revendications

1. Procédé de préparation de plasma sanguin non infectieux, dans lequel on traite le plasma par des agents tensioactifs non ionogènes, on ajoute des lipides biologiquement compatibles, on isole la phase lipidique et on élimine les agents tensioactifs non ionogènes par une extraction en phase solide sur des matières hydrophobes.

2. Procédé suivant la revendication 1, dans lequel le plasma sanguin congelé est dégelé de manière ménagée dans un bain d'eau à 20 jusqu'à 30°C.

3. Procédé suivant l'une des revendications 1 et 2, dans lequel la valeur du pH du plasma est ajustée à un pH de 7,0 à 7,4.

4. Procédé suivant l'une des revendications 1 à 3, dans lequel on met en oeuvre, comme agents tensioactifs non ionogènes, du phosphate de tri-N-butyle (TNBP) et/ou du Triton® X-100.

5. Procédé suivant l'une des revendications 1 à 4, dans lequel on met en oeuvre un mélange aqueux de TNBP et de Triton® X-100.

6. Procédé suivant la revendication 5, dans lequel les proportions du mélange sont TNBP/Triton® X-100/eau = 0,5 à 2/0,5 à 2/2 à 4.

7. Procédé suivant l'une des revendications 1 à 6, dans lequel la phase lipidique est isolée par centrifugation.

8. Procédé suivant l'une des revendications 1 à 7, dans lequel la quantité de lipides est d'environ 10 % (v/v).

9. Procédé suivant l'une des revendications 1 à 8, dans lequel l'extraction en phase solide est effectuée avec des matières pour la chromatographie à phases inversées (reversed phase).

10. Procédé suivant l'une des revendications 1 à 9, dans lequel on utilise des matières dérivées par de l'octadécyle pour la chromatographie à phases inversées.

11. Procédé suivant l'une des revendications 1 à 9, dans lequel l'extraction en phase solide est effectuée par chromatographie à phases inversées à C-18.

12. Procédé suivant l'une des revendications 1 à 11, dans lequel une filtration stérile éventuellement est effectuée à la suite de l'extraction en phase solide après contrôle de la valeur du pH et ajustement à un pH de 7,0 à 7,4.

13. Procédé suivant l'une des revendications 1 à 11, dans lequel le plasma sanguin est lyophilisé, éventuellement en présence d'agents auxiliaires courants pour la lyophilisation.
